# EUROPEAN PATENT APPLICATION

(11) **EP 1 917 928 A1**
(43) Date of publication of application: **07.05.2008**
(21) Application number: 06022613.1
(22) Date of filing: 30.10.2006
(51) Int. Cl.: A61B 19/00

(54) **Clamping apparatus**

(71) Applicant: Nucletron B.V., 3905 TH Veenendaal (NL)
(72) Inventor: Buijs, Emil Matthijs, 3905 GT Veenendaal (NL)
(74) Representative: Valkonet, Rutger

(57) **Abstract**

The invention relates to a clamping apparatus for orientating a medical tool relative to a patient's body comprising: a mounting element arranged to be connected to a support, like an operating table, and a clamping element connected to said mounting element and arranged in accommodating said medical tool in a clamping fashion.

A drawback of the known apparatus are the significant number of different parts as well as the complex adjustability of the medical tool relative to the patients body. The present invention tries to overcome the above identified drawbacks and to provide a clamping apparatus according to the above preamble having significantly lesser component and which allows an easy adjusting of the medical tool relative to the patients body.

To this end the clamping apparatus according to the invention is characterized in that said mounting element is composed of two mounting parts, which are hingedly connected to each other using a longitudinal extending fixating axle. With this construction a medical tool can be secularly fixated within the clamping apparatus enhance easily be oriented relative to a patients body. Furthermore the use of two mounting elements which hingedly connected to each other allows an easy adjustment with a reduced number of component parts.

## Description

The invention relates to a clamping apparatus for orientating a medical tool relative to a patient's body comprising:
a mounting element arranged to be connected to a support, like an operating table, and
a clamping element connected to said mounting element and arranged in accommodating said medical tool in a clamping fashion.

Such apparatus is for example disclosed in US 2002/0038117, where it is used to position a medical tool, here a prostate treatment template with respect to a patients body. Such a template is in particular used for the accurate insertion of multiple hollow catheter needles inside the patients body towards a specific location for performing radiation treatment therapy and therefor an accurate orientation of said template relative to the patient is required.

A drawback of the known apparatus are the significant number of different parts as well as the complex adjustability of the medical tool relative to the patients body. The present invention tries to overcome the above identified drawbacks and to provide a clamping apparatus according to the above preamble having significantly lesser component and which allows an easy adjusting of the medical tool relative to the patients body.

To this end the clamping apparatus according to the invention is characterized in that said mounting element is composed of two mounting parts, which are hingedly connected to each other using a longitudinal extending fixating axle. With this construction a medical tool can be secularly fixated within the clamping apparatus enhance easily be oriented relative to a patients body. Furthermore the use of two mounting elements which hingedly connected to each other allows an easy adjustment with a reduced number of component parts.

Furthermore said fixating axle can be locked in said mounting parts using a locking screw.

In a further preferred embodiment the clamping apparatus according to the invention is characterized in that the clamping element comprises a ball shaped part, which ball shaped part can be accommodated in corresponding concave shaped indents present in each mounting part. This allows an advantageous orienting of a medical tool in regardless nearly any position relative to the patients body. More in particularly according to the invention the clamping element comprises two clamping parts for accommodating said medical tool there between in a clamping fashion, which clamping parts can be urged from and towards each other using a screw element.

In order to obtain a clamping apparatus which is multi functional and can be used for multiple, different medical tools, in a specific embodiment, each clamping part is arranged in accommodating a medical tool specific insertion part. With this feature it is possible to use the clamping apparatus according to the invention for multiple medical tools having different dimensions, without the necessarity to construct tool specific clamping elements. This will result in a simplified construction which is furthermore less expensive.

More particularly both interacting medical tool specific insertion parts form an insert bore for accommodating said medical tool, wherein furthermore the inner dimensions of said insert bore correspond to the outer dimensions of said medical tool. This further enhances the use of the clamping apparatus according to the invention whit multiple, different medical tools having specific unique dimensions.

In a further preferred embodiment each clamping part and each insert part is provided with interacting connecting means, wherein in a specific embodiment the interacting connecting means comprise a pen-hole connection, whereas in an other specific embodiment the interacting connecting means comprise a snap connection.

In order to allow a simplified mounting of the clamping apparatus on a support according to the invention the mounting element comprises a support element arranged to be connected to the support, wherein said support element can be accommodated around said mounting element. In a more specific embodiment the clamping apparatus according to the invention is characterized in that said support element comprises a sleeve part for accommodating said mounting element. The above features allow a simple yet trustworthy placement of the clamping apparatus to a support, resulting in a more stable and safer handling of the clamping apparatus and thus in a more reliable orientating of the medical tool in any position relative to the patients body.

Furthermore in an advantageous embodiment the clamping apparatus according to the invention is manufactured from a non-magnetic material.

More particularly said clamping apparatus is manufactured from aluminium and/or titanium or manufactured from at least polyphenylsulfone.

The invention will now be explained in more detail using the accompanying drawings, which show in:
Figure 1A a first embodiment of a clamping apparatus according to the invention;
Figure 1 B a second embodiment of a clamping apparatus according to the invention;
Figures 2A en 2B detailed views of a mounting element of the clamping apparatus according to the invention;
Figures 3A en 3B detailed views of an embodiment of a clamping element of the clamping apparatus according to the invention;
Figure 4 a detailed view of the support element of the clamping apparatus according to the invention.

For the sake of clarity in the following detailed description the corresponding parts are denoted with identical reference numbers.

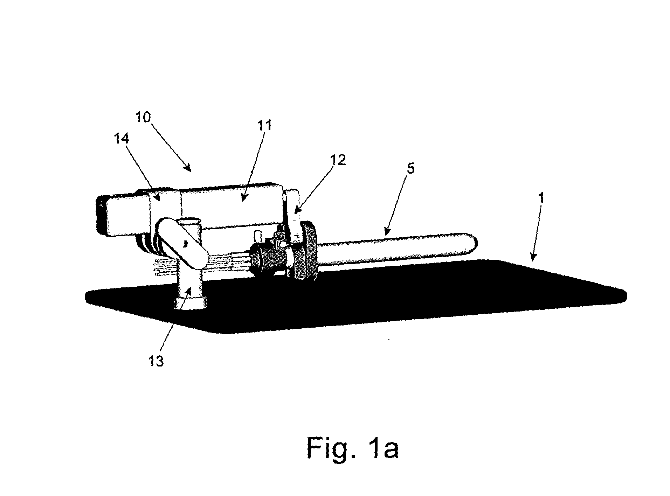

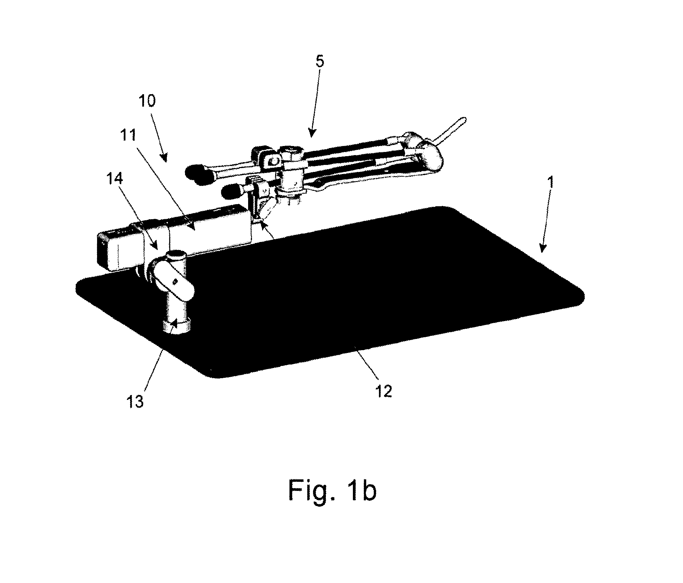

In Figures 1A and 1B, reference no. 10 denotes an embodiment of a clamping apparatus according to the invention. Clamping apparatus 10 is used for orientating a medical tool 5 relative to a patients body (not shown). In Figure 1A the medical tool 5 is embodied as a rectal ultrasound probe bore, which is to be inserted into a patients cavity, for example, the rectum of a man or woman or a woman's vagina for imaging purposes of the internal organs.

The clamping apparatus 10 comprises a mounting element 11, which is arranged to be connected to a support 1, here an operating table, using a support element 13-14. Furthermore the clamping apparatus 10 is provided with a clamping element 12, which is connected to the mounting element 11 and which clamping element is arranged in accommodating the medical tool 5 in a clamping fashion.

In Figure 1B the medical tool 5 is embodied as an orientating tool of a catheter tube for inserting an energy emitting source or for administering a drug at a certain location within the patients body.

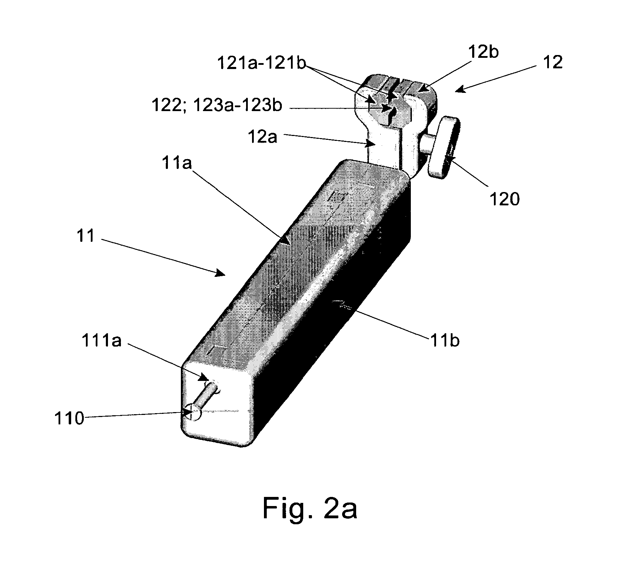

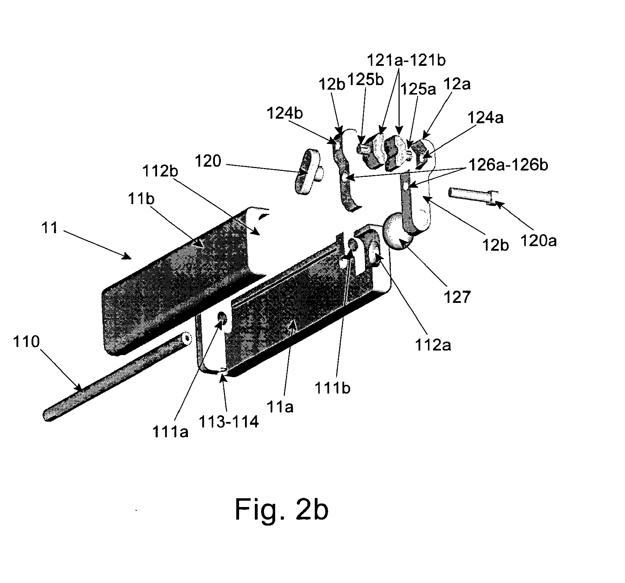

As clearly depicted in Figures 2A and 2B the mounting element 11 is composed of two mounting parts 11A-11B, which are hinghly connected to each other using a longitudinal extending fixating axle 110. To this end the mounting part 11A (see Figure 2B) is provided with flanshes each provided with a true bore 11A-11 B, whereas the second mounting part 11B is in a likewise manner provided with flanges and through bores (not shown) through which the fixating axle 110 can be accommodated. Furthermore each mounting part 11A-11B is provided with concave shaped indents 112A-112B, which functionality will be explained further in the description.

Both mounting parts 11A-11B can be fixed with respect to each other using a locking screw 113 which is accommodated in bore 114 present in mounting part 11A. The locking screw 113 abuts and locks the fixating axle 110 thereby also fixating both mounting parts 11A-11B with each other.

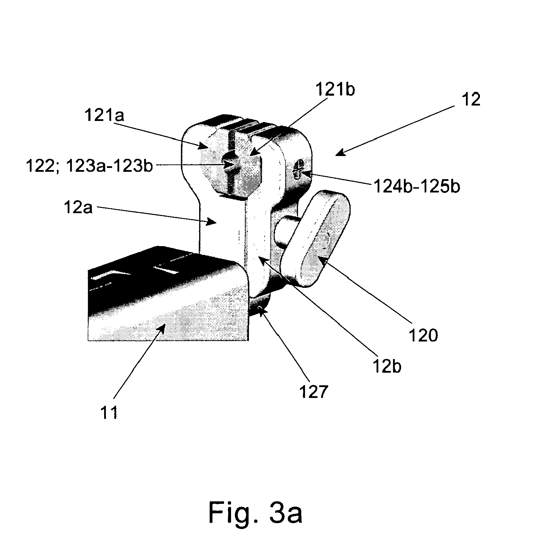

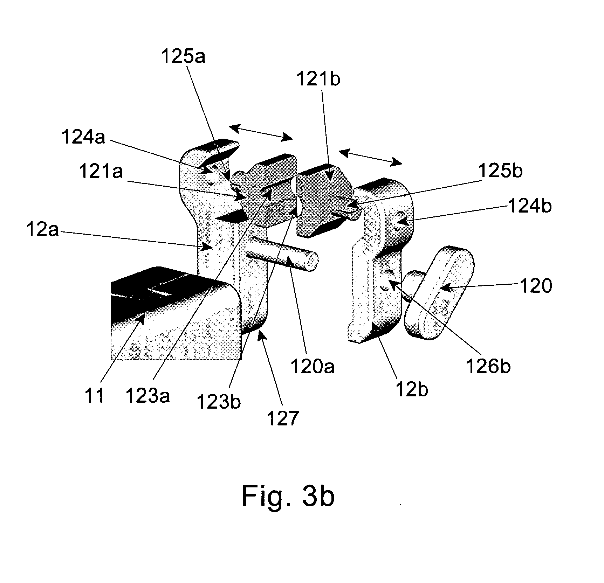

Also depicted in Figures 2A and 2B (and in more detail in Figures 3A and 3B) is the clamping element 12. Clamping element 12 comprises a ball shaped part 127 (see Figure 2B) which can be accommodated in the earlier described concave shaped indents 112A-112B present in both mounting parts 11A-11 B. Accommodating the ball shaped part 127 in the concaved shaped indents of both mounting parts 11A-11B allows an unambiguous orientating of the clamping element 12 with respect to the mounting element 11, which orientation/position can be fixated using the using the locking screw 113, as described previously.

The mounting element 12 furthermore consists of two clamping parts 12A-12B having an arched construction. Both clamping parts 12A-12B can be urged from and towards each other using a screw element 120. Screw element 120 is provided with an internal screw thread which cooperates with an external screw thread provided at the circumferential surface of a screw pin 120A, present on clamping part 12A. Said screw pin 120A can be inserted through a bore 126B, present in clamping part 12B, allowing the mounting of both clamping parts 12A-12B in a clamping fashion using the screw element 120.

According to a further aspect of the invention both clamping parts 12A-12B are used for accommodating a medical tool as depicted in Figures 1A and 1B. In order to obtain a uniform device, which can be used for different specifically designed medical tools, each clamping part 12A-12B is arched shaped and are arranged in accommodating a medical tool specific insert part 121A-121B respectively. Each medical tool specific insert part 121A-121B is provided with a groove 123A-123B which grooves form, when combined together as shown in Figure 2A and 3A, an insert bore 122 for accommodating a specific part of said dedicated medical tool. To this end the inner dimensions of the combined insert bore 122 correspond to the outer dimensions of said specific part of the dedicated medical tool. In order to obtain a proper mounting of the medical tool specific insert parts 121A-121B, both clamping parts 12A-12B and insert part 121A-121B, are provided with interacting connection means 124A-125A; 124B-125B, respectively.

In the embodiment as shown in Figures 2B and 3B, the interacting connecting means 124A-125A and 124B-125B comprises a pen hole connection. However also a quick snap connection can be used for mounting the medical tool specific insert parts 121A-121B in each specific clamping part 12A-12B.

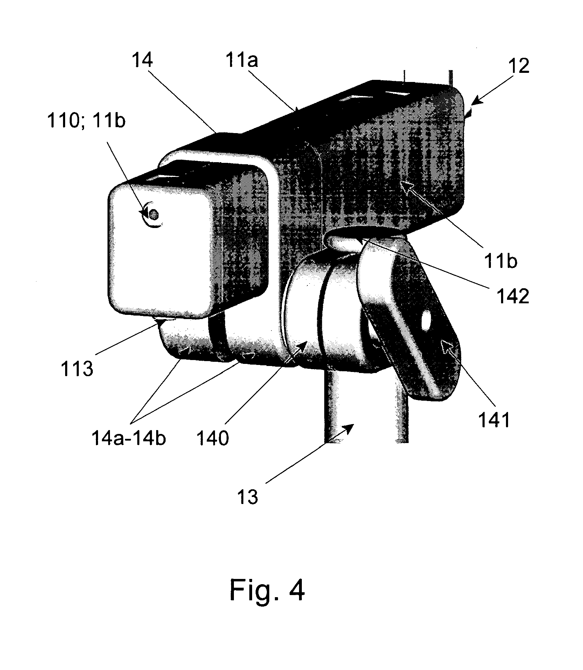

As shown in Figure 7 the mounting element comprises a support element 13-14 which is arranged to be connected to the support 1 (see Figures 1A and 1B). This allows for a proper mounting and orienting of the mounting element 11 with respect to the support and the patients body. The support elements 13-14 can be accommodated around said mounting element 11. To this end the support element comprises a sleeve part 14 which has two sleeve parts ends 14A-14B which accommodate around said mounting element. A corresponding support connector 140 is accommodated around the support 13, which is placed upon the support 1. Although not shown in Figure 4 an support pin is placed through the sleeve part 14 (more particular through the sleeve parts ends 14A-14B), through the additional support element 140 and to a support screw 141 allowing a tight mounting on the support post 13. A further fixation can be created and obtained using a support screw 142.

Preferably the clamping apparatus is made of a non-magnetic material en more particularly manufactured from aluminium and/or titanium or from polyphenylsulfone.

A further advantageous characteristic of the clamping apparatus according to the invention is the fact that it can be made from a material which can be sterilised in order to use the clamping apparatus for multiple times.

The use of a non-magnetic material allows the use of the clamping apparatus according to the invention an CT (computer tomography) and/or MR (magnetic resonance) applications. The advantage of non-magnetic materials result in that the clamping apparatus will not adversely effect the medical application to be performed or administrated.

## Claims

1. Clamping apparatus for orientating a medical tool relative to a patient's body comprising:
a mounting element arranged to be connected to a support, like an operating table, and
a clamping element connected to said mounting element and arranged in accommodating said medical tool in a clamping fashion, **characterized in that** said mounting element is composed of two mounting parts, which are hingedly connected to each other using a longitudinal extending fixating axle.

2. The clamping apparatus according to claim 1, charaterized in that said fixating axle can be locked in said mounting parts using a locking screw.

3. The clamping apparatus according to claim 1 or 2, **characterized in that** the clamping element comprises a ball shaped part, which ball shaped part can be accommodated in corresponding concave shaped indents present in each mounting part.

4. The clamping apparatus according to anyone of the preceding claims, **characterized in that** the clamping element comprises two clamping parts for accommodating said medical tool there between in a clamping fashion, which clamping parts can be urged from and towards each other using a screw element.

5. The clamping apparatus according to claim 4, **characterized in that** each clamping part is arranged in accommodating a medical tool specific insertion part.

6. The clamping apparatus according to claim, **characterized in that** both interacting medical tool specific insertion parts form an insert bore for accommodating said medical tool.

7. The clamping apparatus according to claim 6, **characterized in that** the inner dimensions of said insert bore correspond to the outer dimensions of said medical tool.

8. The clamping apparatus according to anyone of the claims 5-7, **characterized in that** each clamping part and each insert part is provided with interacting connecting means.

9. The clamping apparatus according to claim 8, **characterized in that** the interacting connecting means comprise a pen-hole connection.

10. The clamping apparatus according to claim 8, **characterized in that** the interacting connecting means comprise a snap connection.

11. The clamping apparatus according to anyone of the preceding claims, **characterized in that** the mounting element comprises a support element arranged to be connected to the support, wherein said support element can be accommodated around said mounting element.

12. The clamping apparatus according to claim 11, **characterized in that** said support element comprises a sleeve part for accommodating said mounting element.

13. The clamping apparatus according to anyone of the preceding claims, **characterized in that** the clamping apparatus is manufactured from a non-magnetic material.

14. The clamping apparatus according to claim 13, **characterized in that** the non-magnetic material is at least aluminium.

15. The clamping apparatus according to claim 13 or 14, **characterized in that** the non-magnetic material is at least titanium.

16. The clamping apparatus according to claim 13, **characterized in that** the non-magnetic material is at least polyphenylsulfone.
